# EUROPEAN PATENT APPLICATION

(11) **EP 2 314 201 A1**
(43) Date of publication of application: **27.04.2011**
(21) Application number: 10013783.5
(22) Date of filing: 19.10.2010
(51) Int. Cl.: A61B 3/12, A61B 5/0496

(54) **Apparatus for measuring electrical potential of retina**

(30) Priority: 20.10.2009 JP 2009241074
(71) Applicant: TOMEY CORPORATION, Nagoya-shi Aichi-ken 451-0051 (JP)
(72) Inventor: Funada, Hideaki, Nagoya-shi Aichi-ken 451-0051 (JP)
(74) Representative: Müller-Boré & Partner Patentanwälte

(57) **Abstract**

An apparatus for measuring an electrical potential of retina is provided which is capable of reducing a noise signal in measuring an electrical potential of retina by using electrodes of eyeball-non-contact type and accurately measuring an electrical potential of retina. Fluctuation in electrical potential of retina occurring due to a light stimulus given to one of the left and right eyes of a subject is detected by a corresponding electrode of eyeball-non-contact type, and at the same time, a potential of the other eye that is not light-stimulated is detected by a corresponding electrode of eyeball-non-contact type. Then, a difference between two potential detection signals detected at the same time is found and obtained as a recording signal of electrical potential of retina. Based on this recording signal of electrical potential of retina, a waveform of electrical potential of retina of the light-stimulated eye is found and recorded as an electroretinogram.

## Description

### BACKGROUND OF THE INVENTION

### Field of the Invention

The present invention relates to an apparatus for measuring an electrical potential of retina, and more particularly to an apparatus for measuring an electrical potential of retina, which fluctuates in eyes to which a light stimulus was given by irradiated or illuminated light, by electrodes.

### Description of the Related Art

It has been conventionally known that the human eye, in particular, the retina, has a certain potential (electrical potential of retina) and the potential varies depending on a light stimulus. Such a phenomenon is widely used for examinations, studies, and others. For example, fluctuations in potential are plotted in a diagram with time course shown in the horizontal axis to obtain a so-called electroretinogram (ERG). By using this electroretinogram, electrophysiological examinations on the retina or vision are performed.

Conventionally, to record an electroretinogram (ERG), for example, as disclosed in JP-A-8-154897, JP-A-8-140951, and JP-B-3-23047, electrodes are mounted so as to make contact with corneas and conjunctivas of eyes of a subject, and a light stimulus is given to the retinas, and then potential signals detected by the electrodes in contact with the corneas and conjunctivas are repeatedly measured and recorded to obtain waveforms of electrical potential of retinas. For the light stimulus, a light source having a mechanism of light irradiation on a dome-like inner surface or a corneal electrode having a light-emitting diode incorporated therein is used, thereby stimulating both of the left and right eyes of the subject at the same time to record electrical potential of retinas.

However, in the conventional method, since the electrodes are directly mounted on the eyes, there is a danger of damaging the corneas. Moreover, for subjects who are not allowed to have an electrode to be mounted on their cornea, such as patients with damaged corneas, electrical potential of retinas cannot be measured. Still further, the electrodes used for a patient with an infection are sterilized or thrown away, which poses problems of, for example, making a measuring operation burdensome and increasing cost of measurement.

Meanwhile, as a method of measuring an electrical potential of retinas without making the electrodes in contact with the corneas and mucous membrane of the eyes of the subject, a method has been proposed in which electrodes are affixed on a skin near the respective eyes to measure the electrical potential of retinas. However, since the electrical potentials (voltage) detected by the electrodes are very low, an influence due to mixture of noise is large, thereby disadvantageously distorting the obtained electroretinogram. For this reason, in general, it is difficult to adopt this method.

Specifically, since the electrodes are not mounted directly on the eyes, the above-described measuring method of recording an electroretinogram from the signals of electrical potential of retina detected by the electrodes provided on the skin near the eyes has features of, for example, neither damaging the corneas nor posing a danger of infection. However, the electrical potential of retina detected from the skin near the eye is extremely small, i.e., about 1/5 to 1/10 of the electrical potential of retina detected on the cornea, and it is therefore difficult to identify a waveform of electrical potential of retina. Moreover, when various noises such as an electromyogram or the like occurring due to eye movement or nictitation are mixed, the method easily affected by such a noise signal, thereby disadvantageously making it impossible to correctly record an ERG.

### SUMMARY OF THE INVENTION

The present invention has been made in the situations described above. It is therefore an object of the present invention to provide an apparatus for measuring an electrical potential of retina capable of reducing a noise signal in measuring an electrical potential of retina by using electrodes of eyeball-non-contact type and accurately measuring an electrical potential of retina.

In order to solve the above-described problems or problems recognized from description of the entire specification or the drawings, the present invention can be advantageously carried out in various modes as described below, and the respective modes described below can also be employed in any combination. It is to be understood that the modes or technical features of the present invention can be recognizable based on the inventive concept disclosed in the description of the entire specification and the drawings without being limited to the details of the following description.

(1) An apparatus for measuring an electrical potential of retina, which fluctuates due to light stimulation, the apparatus comprising:
   detecting means of electrical potential of retina which include two electrodes each disposed on a skin near left and right eyes of a subject and which is configured to detect an electrical potential of retina of the left and right eyes corresponding to the two electrodes;
   light stimulating means configured to selectively give a light stimulus to the left and right eyes;
   computing means subtracting, from a detection signal of electrical potential of retina obtained at one of the electrodes of the detecting means of electrical potential of retina corresponding to one of the eyes that is light-stimulated by the light stimulating means, a potential detection signal obtained at the other one of the electrodes of the detecting means of electrical potential of retina corresponding to the other one of the eyes that is not light-stimulated by the light stimulating means, thereby obtaining a recording signal of electrical potential of retina of the eye that is light-stimulated by the light stimulating means; and
   control means selecting one of the eyes to be light-stimulated by the light stimulating means so that no light stimulus is given to the other eye, and based on the recording signal of electrical potential of retina obtained at the computing means, finding a waveform of electrical potential of retina of the light-stimulated eye for recording as an electroretinogram.
(2) The apparatus for measuring an electrical potential of retina according to the above aspect (1), wherein the light stimulating means is configured to alternately give a light stimulus to the left and right eyes, and the control means is configured to find a plurality of waveforms of electrical potential of retina for each of the left and right eyes by a plurality of light stimuli to the left and right eyes and average the plurality of waveforms of electrical potential of retina to find the electroretinogram for each of the left and right eyes.
(3) The apparatus for measuring an electrical potential of retina according to the above aspect (1) or (2), further comprising display means displaying the electroretinogram for each of the left and right eyes recorded in the control means.
(4) The apparatus for measuring an electrical potential of retina according to any one of the above aspects (1) to (3), wherein the two electrodes of the detecting means of electrical potential of retina are each disposed on a skin of a lower eyelid of each of the left and right eyes.
(5) The apparatus for measuring an electrical potential of retina according to any one of the above aspects (1) to (4), further comprising a polarity inversion circuit inverting a polarity of one of the two recording signals of electrical potential of retina for the left and right eyes obtained by the computing means.
(6) The apparatus for measuring an electrical potential of retina according to any one of the above aspects (1) to (5), further comprising:
   reference potential detecting means for obtaining a reference potential of the subject; and
   signal separating means subtracting a reference potential signal obtained by the reference potential detecting means from each of two potential detection signals for the left and right eyes obtained by the detecting means of electrical potential of retina and outputting resultant signals as individual potential signals for the eyes,
   wherein the computing means is configured to find a recording signal of electrical potential of retina of the eye that is light-stimulated by the light stimulating means based on the individual potential signals of the left and right eyes output from the signal separating means.
(7) The apparatus for measuring an electrical potential of retina according to any one of the above aspects (1) to (6), further comprising:
   reference potential detecting means for obtaining a reference potential of the subject; and
   signal separating means subtracting a reference potential signal obtained by the reference potential detecting means from each of two potential detection signals for the left and right eyes obtained by the detecting means of electrical potential of retina and outputting resultant signals as individual potential signals for the eyes,
   wherein the computing means is integrally incorporated in the control means, and in the control means, a differential process is performed on the two individual potential signals output from the signal separating means to find the waveform of electrical potential of retina.
(8) The apparatus for measuring an electrical potential of retina according to the above aspect (6) or (7), wherein the control means includes a first waveform recording memory in which a first waveform based on the individual potential signal for the left eye is recorded and a second waveform recording memory in which a second waveform based on the individual potential signal for the right eye is recorded, at the time of light stimulation to the left and right eyes, and includes a memory, as the computing means, finding a difference between recorded waveforms in the first and second waveform recording memories to obtain and record one of the recording signal of electrical potential of retina and the waveform of electrical potential of retina.
(9) The apparatus for measuring an electrical potential of retina according to the above aspect (8), further comprising display means having a display unit for displaying the waveform recorded in each of the first and second memories of the control means.
(10) The apparatus for measuring an electrical potential of retina according to any one of the above aspects (6) to (9), wherein the reference potential detecting means is constituted by an electrode disposed at a central part of a forehead of the subject, the central part having the same potential as that of each of the left and right eyes.
(11) The apparatus for measuring an electrical potential of retina according to any one of the above aspects (1) to (10), wherein the light stimulating means includes: a left-eye light-emission control circuit for giving a light stimulus to the left eye; and a right-eye light-emission control circuit for giving a light stimulus to the right eye.

As described above, in the apparatus for measuring an electrical potential of retina according to the present invention, fluctuations in electrical potential of retina occurring due to a light stimulus given to one of the left and right eyes of a subject is detected by a corresponding electrode of eyeball-non-contact type, and at the same time, a potential of the other eye that is not light-stimulated is detected by a corresponding electrode of eyeball-non-contact type. Then, a difference between two potential detection signals detected at the same time is found and obtained as a recording signal of electrical potential of retina. Based on this recording signal of electrical potential of retina, a waveform of electrical potential of retina of the light-stimulated eye is found and recorded as an electroretinogram. Thus, a noise signal occurring in accordance with eye movement or nictitation in an electromyogram or the like can be effectively reduced or eliminated to accurately obtain an intended electroretinogram.

That is, in the present invention, even when a noise signal, such as noise due to eye movement, nictitation, or the like and alternating-current noise induced by an alternating-current power supply, is mixed in the detected signal of electrical potential of retina, (retina) potentials of the left and right eye are detected at the same time, and are subjected to a differential process, thereby advantageously eliminating or reducing the mixed noise signal. Thus, a more accurate electroretinogram in which the above-described influence of the noise signal is reduced to the maximum extent possible can be obtained.

Furthermore, in the present invention, since electrodes of eyeball-non-contact type are used as detecting means of electrical potential of retina, an electroretinogram can be easily recorded for patients with a damaged cornea, patients with infections, pediatric patients, and others for which measurement using a cornea-contact electrode for obtaining an electroretinogram is difficult, and also recording of an electroretinogram with less mixture of noise can be provided.

### BRIEF DESCRIPTION OF THE DRAWINGS

The above and other objects, features, advantages and technical and industrial significance of the present invention will be better understood by reading the following detailed description of preferred embodiments of the invention, when considered in connection with the accompanying drawings, in which:
Fig. 1 is a view showing the structure of an embodiment of the apparatus for measuring an electrical potential of retina according to the present invention;
Fig. 2A is a perspective view showing an example of a light stimulating device constituting light stimulating means in the present invention;
Fig. 2B is a cross sectional view of the light stimulating device shown in Fig. 2A;
Fig. 3 is a perspective view showing light-stimulating means in the form of eyeglasses that includes two light stimulating devices shown in Figs. 2A and 2B;
Fig. 4A is a plan view showing an example of an electrode of eyeball-non-contact type for use as an electrode constituting detecting means of electrical potential of retina in the present invention;
Fig. 4B is a cross sectional view taken along line A-A in Fig. 4A;
Fig. 4C is a bottom view of the electrode shown in Fig. 4A;
Fig. 5 is a view showing how a differential process is performed on waveforms (potential signals) obtained by using the apparatus for measuring an electrical potential of retina according to the present invention to obtain a waveform of electrical potential of retina of the light-stimulated eye;
Fig. 6 is a view corresponding to Fig. 1, showing another exemplary embodiment of the apparatus for measuring an electrical potential of retina according to the present invention; and
Fig. 7 is a view showing a variation of the embodiment shown in Fig. 1.

### DETAILED DESCRIPTION OF THE PREFERRED EMBODIMENTS

To further clarify the present invention, there will be described typical embodiments of the present invention in detail with reference to the accompanying drawings.

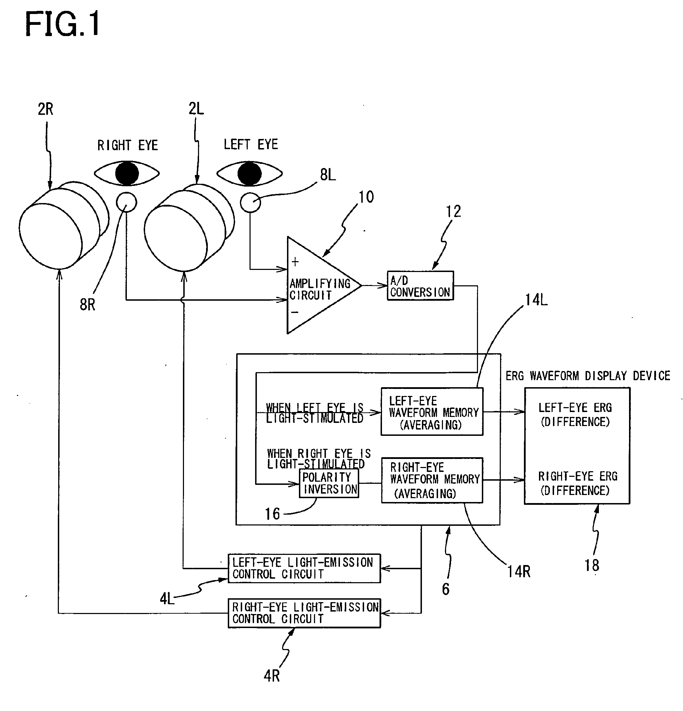

Fig. 1 schematically shows an example of the apparatus for measuring an electrical potential of retina according to the present invention. Here, to give a light stimulus by light emission or irradiation to the left and right eyes of a subject, a left-eye light stimulating device 2L and a right-eye light stimulating device 2R can be disposed before the left and right eyes, respectively. These left and right light stimulating devices 2L and 2R are controlled to have the appropriate amount of stimulating light by adjusting intensity and timing of irradiating light by a left-eye light-emission control circuit 4L and a right-eye light-emission control circuit 4R, respectively. That is, here, with a light-emission control signal output from a control device (CPU circuit) 6, light emission is selectively controlled via the left-eye light-emission control circuit 4L and the right-eye light emission control circuit 4R so that the left-eye light stimulating device 2L and the right-eye light stimulating device 2R alternately emit light, thereby alternately light-stimulating the left and right eyes of the subject.

Further, to detect an electrical potential of retina of each of the left and right eyes of the subject, a left-eye electrode device 8L and a right-eye electrode device 8R are separately disposed on a skin near the left and right eyes, respectively, i.e., a skin of a lower eyelid of the eye. Potential detection signals detected and output by these two electrode devices 8L and 8R are input to a differential amplifier 10, where a differential process (subtraction process) is performed. Then, after signal amplification is performed there, the potential detection signals are converted to digital signals by an A/D converter 12 for output to the control device 6.

The control device 6 is configured, as described above, to output a selective light-emission control signal to the left-eye light-emission control circuit 4L and the right-eye light-emission control circuit 4R. Further, in the control device 6, a differential signal as a recording signal of electrical potential of retina (hereinafter, referred to as a retina potential recording signal) input from the A/D converter 12 is stored in a left-eye waveform memory 14L when the left eye is light-stimulated, and a plurality of waveform recordings accumulated there are averaged to find a waveform of electrical potential of retina (hereinafter, referred to as a retina potential waveform). This waveform is then recorded as a left-eye electroretinogram. When the right eye is light-stimulated, the polarity of the retina potential recording signal is inverted by a polarity inversion circuit 16 and is then recorded in a right-eye waveform memory 14R, and a plurality of waveform recordings are averaged to find a retina potential waveform. This retina potential waveform is then recorded as a right-eye electroretinogram.

Then, the left-eye electroretinogram and the right-eye electroretinogram obtained in the control device 6 are each displayed on a display 18, and also can be each printed as required as an electroretinogram (ERG) of the eye.

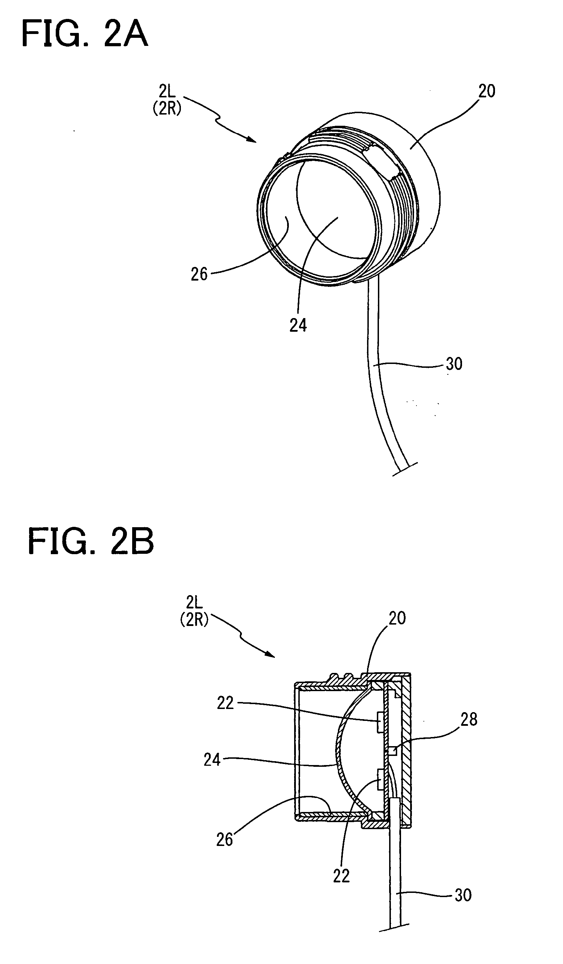

In the above-structured apparatus for measuring an electrical potential of retina according to the present invention, any known component can be selected and used as appropriate as the light-stimulating devices 2L and 2R and the electrode devices 8L and 8R, and examples thereof are shown in Figs. 2A, 2B, 3, 4A, 4B, and 4C.

Among these drawings, Figs. 2A and 2B schematically show the structure of an example of the light stimulating device 2L (2R). In this drawing, a light-stimulation light-emitting diode 22 is incorporated and disposed inside a black closed-bottom cylinder case 20. Light from this light-emitting diode 22 is diffused in a diffusing sphere 24 to form an approximately uniform luminance plane. Furthermore, the light is reflected off a cylindrical light reflective barrel 26 disposed on an inner surface of the closed-bottom cylinder case 20, thereby making the light from the diffusion sphere 24 efficiently irradiated onto the eye. Here, inside the cylinder case 20, a fixation-lamp light-emitting diode 28 is provided so that the subject can fixedly watch the center of the diffusing sphere 24. Further, an LED driving wire 30 is electrically connected to these light-stimulation light-emitting diode 22 and the fixation-lamp light-emitting diode 28, for light-emission driving.

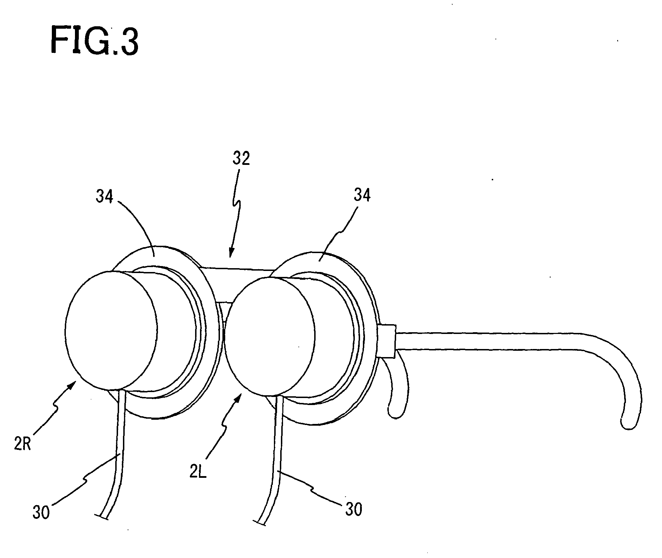

The light stimulating device 2L (2R) structured as shown in Fig. 2A and 2B is mounted on a frame of eyeglasses, as shown in Fig. 3, so as to be able to be worn by the subject. That is, the left-eye light stimulating device 2L and the right-eye light stimulating device 2R are each removably mounted for use on a test lens frame 34 of optometric eyeglasses 32. Thus, the optometric eyeglasses conventionally owned by hospital facilities can be used as they are.

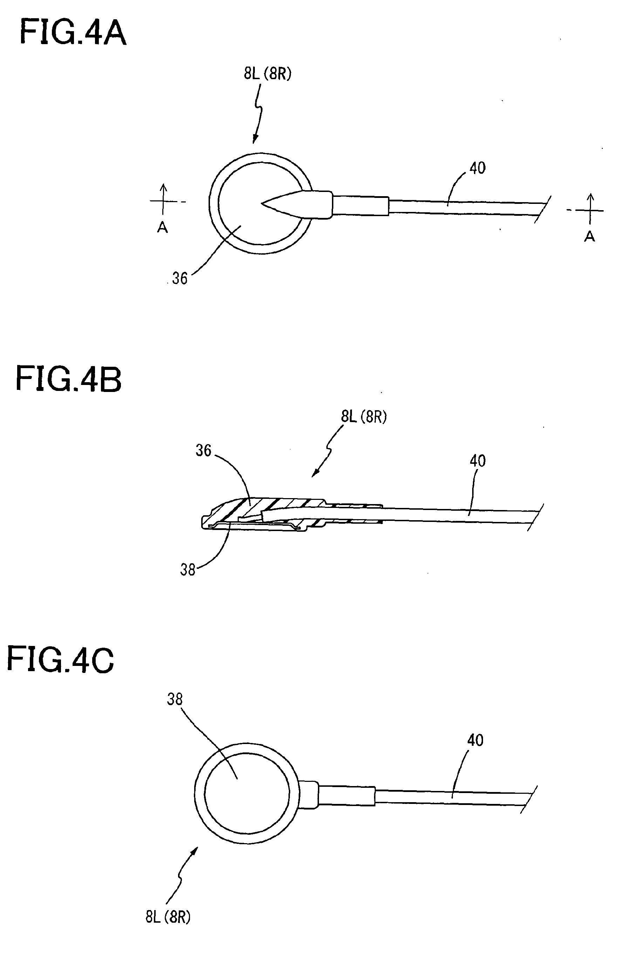

The electrode device 8L (8R) shown in Figs. 4A, 4B, and 4C has a structure in which a disk-shaped electrode plate 38 made of a material such as silver is integrally provided to the lower surface of a disk-shaped support portion. To this electrode plate 38, a signal-leading wire 40 is electrically connected, thereby allowing a potential signal detected on this electrode plate 38 to be taken out through the wire 40. Here, a polarity support member 36 can be manufactured by integral resin molding together with the electrode plate 38 and the signal-leading wire 40. With the use of a conductive electrode paste conventionally known, this electrode device 8L (8R) is adhered correspondingly to the relevant one of the left and right eyes of the subject in a position-fixed manner at a predetermined position on a nearby skin, in general, on a skin of the lower eyelid of the relevant one of the left and right eyes, thereby measuring a potential of the eye.

When measurement is performed for an electroretinogram (ERG) for each of the left and right eyes of the subject by using the apparatus for measuring an electrical potential of retina structured as shown in Fig. 1, the left-eye light stimulating device 2L and the right-eye light stimulating device 2R are first selectively caused to emit light via the left-eye light-emission control circuit 4L and the right-eye light-emission control circuit 4R by a light-emission control signal from the control device 6, thereby selectively giving a light stimulus to the left and right eyes. That is, while a light stimulus is given to one of the left and right eyes, a light stimulus is not given to the other eye. Then, under such a light-stimulation state, potentials of the left and right eyes are obtained at the corresponding two electrode devices 8L and 8R, respectively, and are input to the differential amplifier 10 as potential detection signals. In short, of the left and right eyes, when a light stimulus is given to the left eye, the potential signal obtained at the left-eye electrode device 8L indicates fluctuations over time in electrical potential of retina occurring due to the light stimulus, but various noises are mixed therein, as well known. On the other hand, the potential signal detected at the right-eye electrode device 8R disposed correspondingly to the right eye to which a light stimulus is not given is regarded as a retina signal under a non-light-stimulated state and is also a potential detection signal mixed with various noises, like the detection signal of electrical potential of retina detected at the left-eye electrode device 8L.

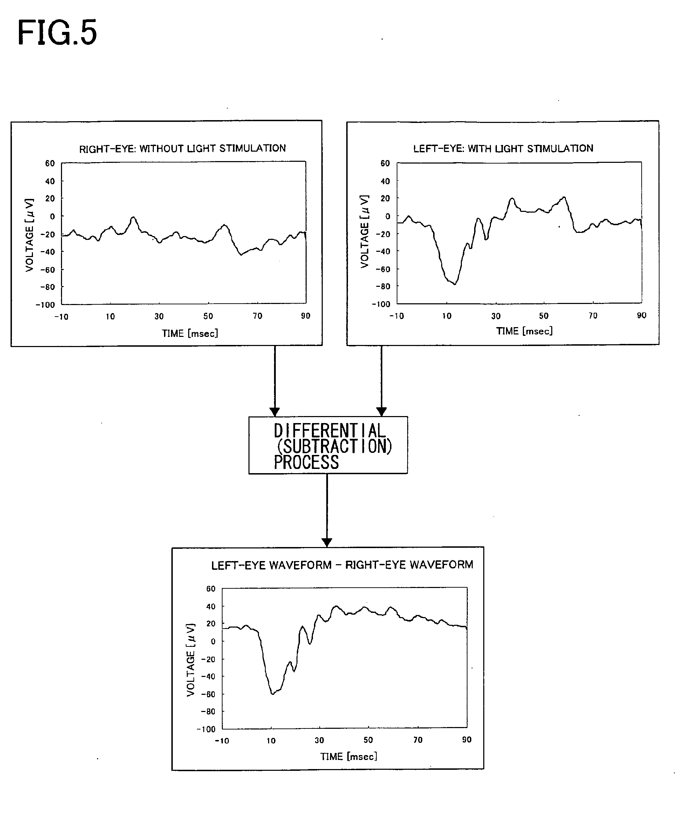

Then, the two potential signals detected at these two electrode devices 8L and 8R are input to the differential amplifier 10 and are subjected to a differential (subtraction) process therein, thereby effectively cancelling the noises mixed in the left and right eyes at the same time. Thus, an intended electroretinogram (ERG) corresponding to the left eye or the right eye can be obtained in a more accurate state. A form of the differential process is shown in Fig. 5. That is, in Fig. 5, a waveform indicating fluctuations over time in electrical potential (voltage) of retina detected at the electrode device 8L in the light-stimulated left eye is shown on an upper right side, and a waveform indicating changes in electrical potential (voltage) of the right eye detected at the electrode device 8R in a non-light-stimulated state is shown on an upper left side. By subtracting the right-eye waveform from the left-eye waveform, a noise-removed electroretinogram (ERG) for the left eye as shown in a graph on a lower portion in Fig. 5 can be obtained. Here, the differential amplifier 10 is used as a computing means that performs subtraction.

Here, light-emission control is alternately performed by the control device 6 over the left-eye light stimulating device 2L and the right-eye light stimulating device 2R. As a result, a light stimulation to the left eye and the right eye of the subject is alternately repeated multiple times, and a plurality of retina potential waveforms for the left eye obtained therethrough are recorded in the left-eye waveform memory 14L, and then these recorded waveforms are averaged. Similarly, for the right eye, a plurality of retina potential waveforms are recorded in the right-eye waveform memory 14R, and then these recorded waveforms are averaged to reduce the amount of noise, thereby more effectively reducing noise mixed in the electroretinogram and more accurately performing measurement for an intended left-eye electroretinogram and an intended right-eye electroretinogram.

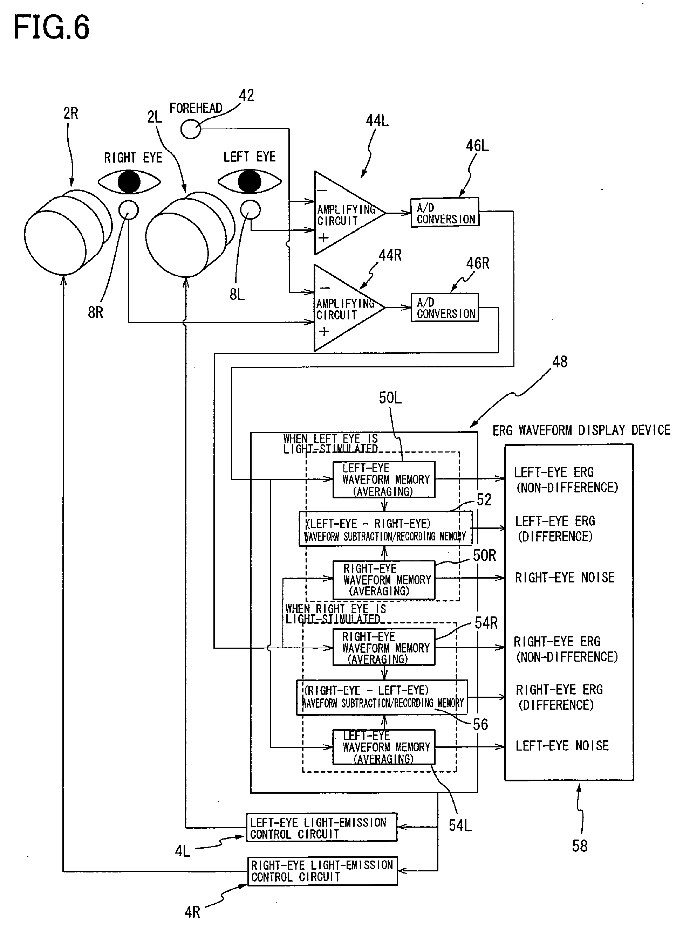

Furthermore, in another example of the apparatus for measuring an electrical potential of retina according to the present invention shown in Fig. 6, a forehead electrode device 42 is provided at the center part of the forehead of the subject, the center part having the same potential with respect to each of the left and right eyes. The potential detected by this forehead electrode device 42 is used as a reference potential. A differential (subtraction) process is performed by differential amplifiers 44L and 44R as the signal separating means in which this reference potential is subtracted from the two potential detection signals of the left and right eyes obtained at the left-eye electrode device 8L and the right-eye electrode device 8R, respectively, and the result is output as an individual potential signal of each of the eyes. Furthermore, these potential signals are converted at a left-eye A/D converter 46L and a right-eye A/D converter 46R to digital signals, and then they are input to a control device 48. Here, the forehead electrode device 42 has a structure similar to that of the above-described left-eye electrode device 8L and right-eye electrode device 8R. As with the example shown in Fig. 1, light-emission of the left-eye light stimulating device 2L and the right-eye light stimulating device 2R are controlled regarding the intensity and timing of light irradiated to the corresponding eye by the left-eye light-emission control circuit 4L and the right-eye light-emission control circuit 4R, respectively, based on a light-emission control signal from the control device 48.

The control device 48 is provided with a left-eye waveform memory 50L, a right-eye waveform memory 50R, and a (left-eye - right-eye) waveform subtraction/recording memory 52, which are used when the left eye is light-stimulated; and a right-eye waveform memory 54R, a left-eye waveform memory 54L, and a (right-eye - left-eye) waveform subtraction/recording memory 56, which are used when the right eye is light-stimulated. An individual potential signal for the relevant eye is input to a corresponding one of the waveform memories 50L and 50R and a corresponding one of the waveform memories 54L and 54R. That is, the individual potential signal obtained by separating, at the differential amplifier 44L, the potential signal detected regarding the left eye is input to the left-eye waveform memory 50L for use in light stimulation to the left eye and the left-eye waveform memory 54L for use in light stimulation to the right eye, and its potential waveform is recorded. Further, a plurality of input waveforms are averaged. The individual potential signal obtained by separating, at the differential amplifier 44R, the potential signal detected regarding the right eye is input to the right-eye waveform memory 50R for use in light stimulation to the left eye and the right-eye waveform memory 54R for use in light stimulation to the right eye, and its potential waveform is recorded. The potential waveforms are also averaged.

Furthermore, when the left eye is light-stimulated, the (left-eye - right-eye) waveform subtraction/recording memory 52 performs a differential (subtraction) process of subtracting the right-eye potential waveform (the potential detection signal including noise and others) recorded in the right-eye waveform memory 50R from the retina potential waveform (the retina potential recording signal) recorded in the left-eye waveform memory 50L, and then records the result therein. When the right eye is light-stimulated, the (right-eye - left-eye) waveform subtraction/recording memory 56 performs a differential (subtraction) process of subtracting the left-eye potential waveform (the potential detection signal including noise and others) recorded in the left-eye waveform memory 54L from the retina potential waveform (the retina potential recording signal) recorded in the right-eye waveform memory 54R, and then records the result therein. That is, the (left-eye - right-eye) waveform subtraction/recording memory 52 and the (right-eye - left-eye) waveform subtraction/recording memory 56 are used as the computing means that performs subtraction.

On the display 58 for the ERG waveforms (retina potential waveforms), correspondingly to the left-eye waveform memory 50L, the (left-eye - right-eye) waveform subtraction/recording memory 52, the right-eye waveform memory 50R, the right-eye waveform memory 54R, the (right-eye - left-eye) waveform subtraction/recording memory 56, and the left-eye waveform memory 54L, which are all provided in the control device 48, a left-eye ERG (non-difference), a left-eye ERG (difference), a right-eye noise, a right-eye ERG (non-difference), a right-eye ERG (difference), and a left-eye noise are displayed as waveforms.

Therefore, in the above-structured apparatus for measuring an electrical potential of retina, when the left eye is light-stimulated, the left-eye ERG (difference) based on an output from the (left-eye - right-eye) waveform subtraction/recording memory 52 is displayed on the display 58. When the right eye is light-stimulated, the right-eye ERG (difference) based on an output from the (right-eye - left-eye) waveform subtraction/recording memory 56 is displayed on the display 58. As a result, an intended electroretinogram can be obtained. If extreme noise is mixed only in a waveform signal detected in a non-light-stimulated eye, in measurement for an intended electroretinogram, by observation of the situation of the right-eye noise or the left-eye noise on the display 58, the left-eye ERG (non-difference) or the right-eye ERG (non-difference) obtained by using a retina potential waveform before the differential (subtraction) process can be adopted as an intended electroretinogram. As a result, a waveform diagram with less mixture of noise can be obtained.

While typical embodiments of the present invention have been described, for illustrative purpose only, it is to be understood that the present invention is not limited to the details of the illustrated embodiments.

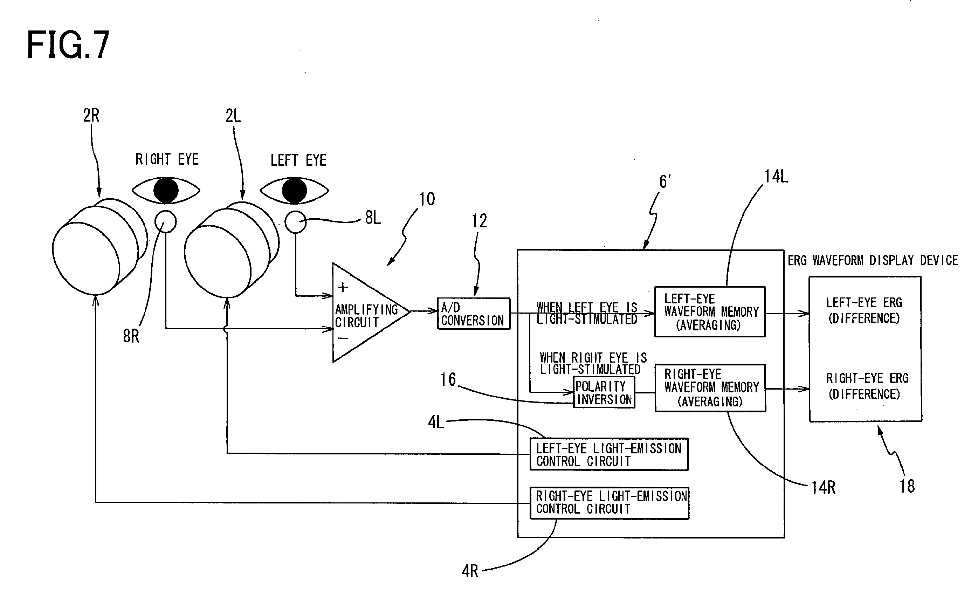

For example, in the apparatus for measuring an electrical potential of retina shown in Fig. 1, the left-eye light-emission control circuit 4L and the right-eye light-emission control circuit 4R are provided separately from the control device 6. Unlike this, as exemplified in Fig. 7, it is possible to adopt the structure in which the left-eye light-emission control circuit 4L and the right-eye light-emission control circuit 4R are integrally provided in a control device 6'. Similarly, also in the apparatus for measuring an electrical potential of retina shown in Fig. 6, it is possible to unify the control device 48, the left-eye light-emission control circuit 4L, and the right-eye light-emission control circuit 4R.

Further, in the apparatus for measuring an electrical potential of retina shown in Fig. 6, based on recorded information in the left-eye waveform memories 50L and 54L and the right-eye waveform memories 50R and 54R, each of which records an individual potential signal of the relevant eye at the time of light stimulation and at the time of no light stimulation, a differential (subtraction) process is performed at the (left-eye - right-eye) waveform subtraction/recording memory 52 and the (right-eye - left-eye) waveform subtraction/recording memory 56, and furthermore the result is recorded as a retina potential waveform. However, instead of the above structure, it is possible to provide a differential (subtraction) circuit similar to the differential amplifier 10 shown in Fig. 1 outside or inside of the control device 48 to record an intended electroretinogram.

Further, as the light stimulating devices 2L and 2R, in addition to the exemplified structures, various known structures can be adopted as appropriate. For example, structures disclosed in JP-A-2001-37720 and JP-A-2004-73807 can be employed.

In addition, in the example shown in Fig. 6, as referential potential detecting means for taking out a reference potential of the subject, one forehead electrode device 42 is provided. However, two electrodes for the left eye and the right eye may be used to detect and take out a reference potential as a reference potential signal and a differential process may be performed between the reference potential signal and the potential detection signal of the corresponding eye. It is to be understood that, in this case, the electrodes are provided each at a position of the body of the subject where the referential potential signals obtained at these two electrodes are almost equal to each other.

Although further details will not be described herein, it is to be understood that the present invention may be embodied with various other changes and modifications which may occur to those skilled in the art, without departing from the spirit and scope of the invention.

## Claims

1. An apparatus for measuring an electrical potential of retina, which fluctuates due to light stimulation, the apparatus comprising:
detecting means of electrical potential of retina (8L, 8R) which include two electrodes (38) each disposed on a skin near left and right eyes of a subject and which is configured to detect an electrical potential of retina of the left and right eyes corresponding to the two electrodes;
light stimulating means (2L, 2R) configured to selectively give a light stimulus to the left and right eyes;
computing means (10, 52, 56) subtracting, from a detection signal of electrical potential of retina obtained at one of the electrodes of the detecting means of electrical potential of retina corresponding to one of the eyes that is light-stimulated by the light stimulating means, a potential detection signal obtained at the other one of the electrodes of the detecting means of electrical potential of retina corresponding to the other one of the eyes that is not light-stimulated by the light stimulating means, thereby obtaining a recording signal of electrical potential of retina of the eye that is light-stimulated by the light stimulating means; and
control means (6, 48) selecting one of the eyes to be light-stimulated by the light stimulating means so that no light stimulus is given to the other eye, and based on the recording signal of electrical potential of retina obtained at the computing means, finding a waveform of electrical potential of retina of the light-stimulated eye for recording as an electroretinogram.

2. The apparatus for measuring an electrical potential of retina according to claim 1, wherein the light stimulating means is configured to alternately give a light stimulus to the left and right eyes, and the control means is configured to find a plurality of waveforms of electrical potential of retina for each of the left and right eyes by a plurality of light stimuli to the left and right eyes and average the plurality of waveforms of electrical potential of retina to find the electroretinogram for each of the left and right eyes.

3. The apparatus for measuring an electrical potential of retina according to claim 1 or 2, further comprising display means (18) displaying the electroretinogram for each of the left and right eyes recorded in the control means.

4. The apparatus for measuring an electrical potential of retina according to any one of claims 1 to 3, wherein the two electrodes of the detecting means of electrical potential of retina are each disposed on a skin of a lower eyelid of each of the left and right eyes.

5. The apparatus for measuring an electrical potential of retina according to any one of claims 1 to 4, further comprising a polarity inversion circuit (16) inverting a polarity of one of the two recording signals of electrical potential of retina for the left and right eyes obtained by the computing means.

6. The apparatus for measuring an electrical potential of retina according to any one of claims 1 to 5, further comprising:
reference potential detecting means (42) for obtaining a reference potential of the subject; and
signal separating means (44L, 44R) subtracting a reference potential signal obtained by the reference potential detecting means from each of two potential detection signals for the left and right eyes obtained by the detecting means of electrical potential of retina and outputting resultant signals as individual potential signals for the eyes,
wherein the computing means (52, 56) is configured to find a recording signal of electrical potential of retina of the eye that is light-stimulated by the light stimulating means based on the individual potential signals of the left and right eyes output from the signal separating means.

7. The apparatus for measuring an electrical potential of retina according to any one of claims 1 to 6, further comprising:
reference potential detecting means (42) for obtaining a reference potential of the subject; and
signal separating means (44L, 44R) subtracting a reference potential signal obtained by the reference potential detecting means from each of two potential detection signals for the left and right eyes obtained by the detecting means of electrical potential of retina and outputting resultant signals as individual potential signals for the eyes,
wherein the computing means (52, 56) is integrally incorporated in the control means, and in the control means, a differential process is performed on the two individual potential signals output from the signal separating means to find the waveform of electrical potential of retina.

8. The apparatus for measuring an electrical potential of retina according to claim 6 or 7, wherein the control means includes a first waveform recording memory (50L, 54L) in which a first waveform based on the individual potential signal for the left eye is recorded and a second waveform recording memory (50R, 54R) in which a second waveform based on the individual potential signal for the right eye is recorded, at the time of light stimulation to the left and right eyes, and includes a memory, as the computing means (52, 56), finding a difference between recorded waveforms in the first and second waveform recording memories to obtain and record one of the recording signal of electrical potential of retina and the waveform of electrical potential of retina.

9. The apparatus for measuring an electrical potential of retina according to claim 8, further comprising display means (58) having a display unit for displaying the waveform recorded in each of the first and second memories of the control means.

10. The apparatus for measuring an electrical potential of retina according to any one of claims 6 to 9, wherein the reference potential detecting means is constituted by an electrode (42) disposed at a central part of a forehead of the subject, the central part having the same potential as that of each of the left and right eyes.

11. The apparatus for measuring an electrical potential of retina according to any one of claims 1 to 10, wherein the light stimulating means includes: a left-eye light-emission control circuit (4L) for giving a light stimulus to the left eye; and a right-eye light-emission control circuit (4R) for giving a light stimulus to the right eye.
